(19) Europäisches Patentamt
European Patent Office
Office européen des brevets

(11) **EP 2 980 716 B1**

(12) **EUROPEAN PATENT SPECIFICATION**

(45) Date of publication and mention
of the grant of the patent:
**02.09.2020  Bulletin 2020/36**

(51) Int Cl.:
**G16H 10/40** *(2018.01)*        **G16H 40/00** *(2018.01)*
**G16H 40/63** *(2018.01)*

(21) Application number: **15178622.5**

(22) Date of filing: **28.07.2015**

(54) **SYSTEM AND METHOD FOR RECOVERING FROM A LARGE OUT-OF-CONTROL CONDITION IN A CLINICAL DIAGNOSTIC PROCESS**

SYSTEM UND VERFAHREN ZUR WIEDERHERSTELLUNG EINER GROSSEN, AUSSER KONTROLLE GERATENEN STÖRUNG IN EINEM KLINISCHEN DIAGNOSEVERFAHREN

SYSTÈME ET PROCÉDÉ PERMETTANT DE RÉCUPÉRER D'UNE SITUATION DE PERTE DE CONTRÔLE IMPORTANTE DANS UN PROCESSUS DE DIAGNOSTIC CLINIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2014  US 201414448336**

(43) Date of publication of application:
**03.02.2016  Bulletin 2016/05**

(73) Proprietor: **Bio-rad Laboratories, Inc.**
**Hercules, CA 94547 (US)**

(72) Inventors:
 • **Kuchipudi, Lakshmi Samyukta**
   **Wylie, TX Texas 75098 (US)**
 • **Parvin, Curtis**
   **McKinney, TX Texas 75070 (US)**
 • **Yundt-Pacheco, John**
   **Fairview, TX Texas 75069 (US)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
   **US-A1- 2005 125 186      US-A1- 2012 330 866**

 • **JAMES M. LUCAS ET AL: "Fast Initial Response for CUSUM Quality-Control Schemes: Give Your CUSUM A Head Start", TECHNOMETRICS., vol. 42, no. 1, 1 February 2000 (2000-02-01), pages 102-107, XP055231278, US ISSN: 0040-1706, DOI: 10.1080/00401706.2000.10485987**
 • **Anonymous: "CUSUM", Wikipedia, 28 May 2014 (2014-05-28), XP055230434, Retrieved from the Internet: URL:https://en.wikipedia.org/w/index.php?title=CUSUM&oldid=610483581 [retrieved on 2015-11-23]**
 • **Anonymous: "Lookup table", Wikipedia, the free encyclopedia, 21 June 2014 (2014-06-21), pages 1-5, XP055211548, Retrieved from the Internet: URL:http://web.archive.org/web/20140621081 859/http://en.wikipedia.org/wiki/Lookup_ta ble [retrieved on 2015-09-07]**
 • **EMMANUEL YASHCHIN: "On the analysis and design of CUSUIVI- Shewhart control schemes", IBM JOURNAL OF RESEARCH AND DEVELOPMENT., vol. 29, no. 4, 1 July 1985 (1985-07-01), pages 377-391, XP055231289, US ISSN: 0018-8646, DOI: 10.1147/rd.294.0377**

**Description**

**CROSS-REFERENCE TO RELATED APPLICATIONS**

Not applicable.

**STATEMENT REGARDING FEDERALLY SPONSORED RESEARCH OR DEVELOPMENT**

Not applicable.

**BACKGROUND OF THE INVENTION**

1. FIELD OF THE INVENTION

The present invention relates to clinical diagnostic processes, and more particularly to a system and method for recovering from a large out-of-control condition in such processes.

2. DESCRIPTION OF RELATED ART

Clinical diagnostic laboratories employ various quality control schemes to control clinical diagnostic processes to ensure the accuracy of diagnostic results. In the United States, Westgard is a well-known scheme, with other schemes, such as RiliBAK, more common outside of the U.S. More recently developed patient-data based schemes, such as the Biometric Quality Control process as described in U.S. Patent No. 8,099,257, are also becoming widely used.

In addition, in US 2005/125186 A1 (which is regarded as the closest prior art), a method and a corresponding system for determining an optimal quality control rule for confirming that an analytical process is in control after an event has occurred, are disclosed. According to the disclosed method, a maximum false rejection rate is identified, before a maximum acceptable risk of producing patient test results containing analytical errors exceeding a specified total allowable error value is identified. Subsequently, a minimum number of quality control samples satisfying the false rejection rate and acceptable risk requirements is determined, before optimal quality control rule rejection limits are determined that satisfy the false rejection rate and acceptable risk requirements within the number of quality control samples.

Also, in US 2012/330866 A1, further methods and corresponding systems for optimizing a quality control strategy are disclosed.

Regardless of the specific quality control (QC) process employed, a common characteristic of known clinical diagnostic processes is that eventually all such processes will fail in some manner. Those failures can be generally divided into two categories: stop failures, in which the entire testing system or process is halted; and run failures, in which the testing system or process continues, but is producing potentially erroneous results. A failure in which the clinical diagnostic process continues to run is commonly referred to as an out-of-control condition - i.e., the diagnostic process is still operating, but is not operating within desired parameters. An out-of-control condition affects all of the specimens evaluated while the out-of-control condition exists, namely in the form of increased amount of measurement error in the evaluation. Only when the measurement error in a result exceeds a predetermined level (i.e., an allowable total error $TE_a$) is the result deemed to be unsuitable or unreliable. If the out-of-control condition is small, it may render only a fraction of the specimens evaluated during the existence of the condition to be unreliable. However, if the out-of-control condition is large, it may render all specimens evaluated during the existence of the condition to be unreliable.

Out-of-control conditions in clinical diagnostic processes can remain undetected for long periods of time. Because the patient specimens being analyzed themselves have unknown analytical concentrations, it is difficult to determine from those specimens whether their quality has been compromised. Thus, when an out-of-control condition is finally detected, a laboratory is obligated to stop the testing process to identify the results which are deemed unreliable and to take corrective action to remedy the out-of control condition so that accurate and reliable results may be resumed.

One method of ensuring that accurate and reliable results are provided is to first correct the out-of-control condition, and then retest all of the patient specimens that were tested since the last known "good" state of the clinical diagnostic process. This typically is identified as the last time the quality control process has been evaluated and deemed "good". While this method ensures that most, if not all potential unreliable results are accounted for and the specimens retested, it can be overly burdensome and onerous. For example, if one-thousand specimens have been tested since the last known good condition, and an out-of-control error occurs just after specimen nine-hundred and ninety-nine, then all one thousand specimens would be retested when only the one-thousandth specimen is actually unreliable. If the time of occurrence of the out-of-control condition can be determined accurately, then the unreliable results can be identified and re-testing of specimens can be reduced.

Thus, it is apparent that current quality control processes relying on identifying large out-of-control conditions and simply retesting all specimens is insufficient, often leading to duplicative testing and unnecessary retesting of specimens.

## BRIEF SUMMARY OF THE INVENTION

In order to solve the above-referenced problem, a method for analyzing a specimen, a corresponding system and a corresponding non-transitory computer-readable medium as defined in the independent claims are provided with the present invention. Further preferred embodiments of the present invention are defined in the depending claims. In particular, the present invention is directed to a system and method for identifying and recovering from large out-of-control conditions in a clinical diagnostic process. The system and method of the present invention automatically characterizes large out-of-control conditions and predicts the number of incorrect patient specimen results since the last known good quality control evaluation. When the out-of-control condition is resolved, the system and method retests patient specimen until the point of failure is identified using a defined CUSUM rule. Corrections to specimen data are thus required only when warranted.

In one aspect, the system and method of the present invention provides automated identification of a large out-of-control condition in a laboratory quality control (QC) process. In another aspect, the system and method of the present invention estimates the expected number of incorrect results evaluated since the last good QC event by estimating the magnitude of the out-of control condition and constructing an allowable total error ($TE_a$) profile to estimate the probability of a specimen result being incorrect. In yet another aspect, the system and method of the present invention automatically designs a CUSUM rule which is applied to determine whether a re-evaluated specimen result is accurate and to thus detect the origination point of the large out-of-control condition. In another aspect, the system and method of the present invention automatically determines which reevaluated patient specimens need to be corrected when the results have already been reported or published.

The system and method of the present invention will be described herein in conjunction with the Biometric Quality Control process of U.S. Patent No. 8,099,257. However, it should be understood that the system and method of the present invention may equally be used with any other quality control process, biometric or otherwise, such implementations are contemplated by and within the scope of the present invention.

Reference to the remaining portions of the specification, including the drawings and claims, will realize other features and advantages of the present invention. Further features and advantages of the present invention, as well as the structure and operation of various embodiments of the present invention, are described in detail below with respect to the accompanying drawings and claims. In the drawings, like reference numbers indicate identical or functionally similar elements.

## BRIEF DESCRIPTION OF THE DRAWINGS

The present invention will be described in greater detail in the following detailed description of the invention with reference to the accompanying drawings that form a part hereof, in which:

FIG. 1 depicts a block diagram of a client computer system configured with an application module for detecting large out-of-control conditions in a clinical diagnostic process according to a first exemplary embodiment of the present invention.
FIG. 2 depicts a block diagram of a network arrangement for executing a shared application and/or communicating data and commands between multiple computing systems and devices according to an exemplary embodiment of the present invention.
FIG. 3A depicts a first portion of a flow diagram of a process for detecting large out-of-control conditions in a clinical diagnostic process according to an exemplary embodiment of the present invention.
FIG. 3B depicts a second portion of a flow diagram of a process for detecting large out-of-control conditions in a clinical diagnostic process according to an exemplary embodiment of the present invention.

## DETAILED DESCRIPTION OF EXEMPLARY EMBODIMENTS

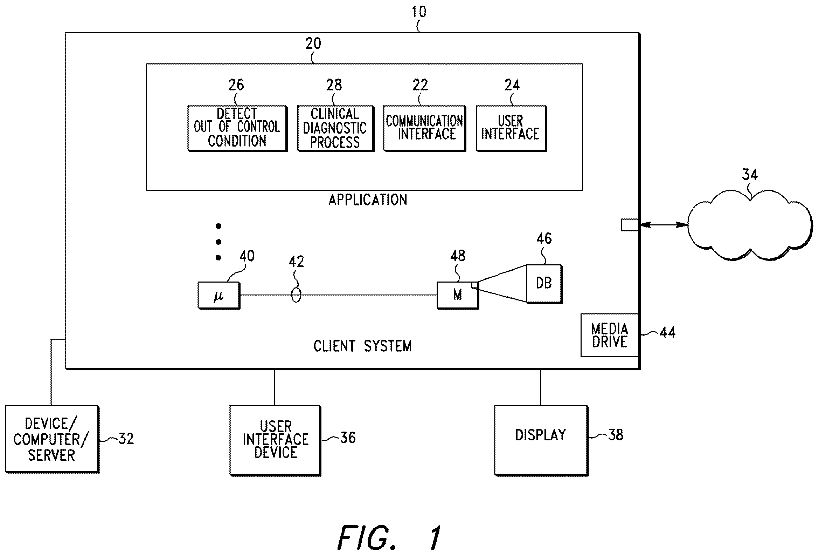

A system and method for automatically recovering from a large out-of-control condition in a clinical diagnostic process in accordance with exemplary embodiments of the present invention are depicted in FIGS 1 through 3B. While the invention will be described in detail hereinbelow with reference to the depicted exemplary embodiments and alternative embodiments, it should be understood that the invention is not limited to the specific configurations shown and described in these embodiments. Rather, one skilled in the art will appreciate that a variety of configurations may be implemented in accordance with the present invention.

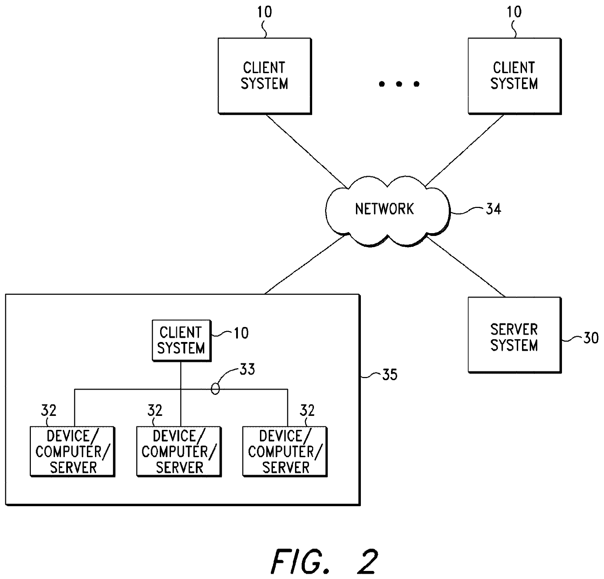

Looking first to FIGS. 1 and 2, a system client computer system (e.g., a clinical diagnostic instrument) 10 is configured with an application module 20 operable to perform testing on various analytes, such as patient specimens or quality control specimens. Application module 20 may execute any sequence of diagnostic steps or one or more diagnostic algorithms in conjunction with implementing any clinical diagnostic process, such as a hematology analyzer or any other clinical diagnostic or analytical process. As best shown in FIG. 2, a plurality of client computer systems 10 may be arranged in a network configuration for executing a shared application and/or for communicating data and commands between multiple computing systems and devices according to an exemplary embodiment of the present invention. It should be understood that client computer system 10 may operate as a stand-alone system such as a diagnostic instrument device or laboratory instrument, or it may be connected to a server system 30 and/or other client systems 10 and/or other devices/servers 32 over a network 34.

Several elements in the system depicted in FIGS. 1 and 2 are well-known, existing elements and variations of those exemplary elements may be implemented in accordance with the present invention. For example, client system 10 may include a desktop personal computer, a workstation, a laptop computer, a handheld mobile device, or any other computing device capable of executing the application module 20. In client-server or networked embodiments, client system 10 is configured to interface directly or indirectly with server system 30 over network 34. Network 34 may be any type of network known in the art, such as a local area network (LAN), a wide area network (WAN), the Internet, an ad-hoc network, or any other type of network. Client system 10 may also communicate directly or indirectly with one or more other client systems 10 and devices/servers 32 over network 34. Client system 10 preferably executes a web browsing program, such as Microsoft's Internet Explorer, Netscape Navigator, Opera or the like, allowing a user of client system 10 to access, process and view information and pages available to it from server system 30 or other server systems over network 34. Client system 10 also preferably includes one or more user interface devices 36, such as a keyboard, a mouse, a touch screen, graphical tablet, pen or the like, for interacting with a graphical user interface (GUI) provided on a display 38. Display 38 is preferably a monitor or LCD screen, but may be any type of display device known in the art.

In one exemplary embodiment, application module 20 executes entirely on client system 10 (e.g., stand-alone), however, in alternative embodiments the application module may be executed in a networked environment such as a client-server, peer-to-peer, or multi-computer networked environment where portions of the application code may be executed on different portions of the network system or where data and commands are exchanged between various components or devices executing portions of the application code. In local network embodiments, interconnection via a LAN is preferred, however, it should be understood that other networks can be used, such as the Internet or any intranet, extranet, virtual private network (VPN), non-TCP/IP based network, WAN or the like. For example, in the exemplary embodiment depicted in FIG. 2, a LAN 33 interconnects multiple devices to a client system 10. Such a network is exemplary of a multiple instrument environment 35, such as a laboratory or hospital, where multiple instruments, devices, or servers are connected to a client system 10 in a Laboratory Information System (LIS) arrangement. LAN 33 may include wireless and wired links and nodes, and use various communication protocols as are well known in the art.

Preferably, server system 30 acts as a central computer system that executes a majority of, or all, of the application module code, with each client system 10 acting as a terminal or log-in point for a user. For example, client system 10 may reside in a laboratory or a hospital multiple instrument environment 35 as part of a LIS, while server system 30 may reside in a geographically remote location. In such a configuration, the application module code is preferably executed entirely on server system 30, with data and commands sent between client system 10 over network 34. For example, if client system 10 resides in a laboratory, client system 10 would provide the required patient data and/or test results/data, and other information from a local database and local instruments and devices for processing by server system 30, which would then provide processing results back to client system 10, or to other computer systems. It should be understood that the application code may execute entirely on a single system or portions may execute on both systems 10 and 30 (or on multiple systems in other exemplary embodiments) as desired for computational efficiency purposes. Additionally, a client system 10 in a multiple instrument environment 35 may execute a portion or all of the application module code.

Looking again to FIG. 1, in an exemplary embodiment, client system 10 and some or all of its components are operator configurable through operation of the application module 20, which includes computer code executable on a central processing unit 40 coupled to other components over one or more busses 42 as is well known in the art. Computer code, including instructions for operating and configuring client system 10 (or other systems on which the application module is executing, such as server system 30 of FIG. 2) to process data content, monitor and control application processes, and render GUI images as described herein, is preferably stored on a hard disk, but the entire program code, or portions thereof, may also be stored in any other volatile or non-volatile memory medium or device as is well known, such as a ROM or RAM, or provided on any media capable of storing program code, such as a compact disk (CD) medium, digital versatile disk (DVD) medium, a floppy disk, and the like.

An appropriate media drive 44 is provided for receiving and reading documents, data and code from such a computer-readable medium. Additionally, the entire program code of module 20, or portions thereof, or related commands such as Active X commands, may be transmitted and downloaded from a software source, such as server system 30, to client system 10 or from another server system or computing device to client system 10 over the Internet as is well known, or transmitted over any other conventional network connection (e.g., extranet, VPN, LAN, etc.) using any communication medium and protocols (e.g., TCP/IP, HTTP, HTTPS, Ethernet, etc.) as are also well known. It should be understood that computer code for implementing aspects of the present invention can be implemented in a variety of coding languages such as C, C++, Java, Visual Basic, and others, or any scripting language, such as VBScript, JavaScript, Perl or markup languages such as XML, that can be executed on client system 10 and/or in a client server or networked arrangement. In addition, a variety of languages can be used in the external and internal storage of data, e.g., patient results, device and instrument information (e.g., IDs, date/time stamps, calibration information, temperature information, etc.), and other information, according to aspects of the present invention.

In an exemplary embodiment, application module 20 includes instructions for monitoring and controlling clinical diagnostic processes, as well as for providing user interface configuration capabilities, as described herein. Application module 20 is preferably downloaded and stored on media hard drive 44 (or other memory such as a local or attached RAM or ROM), although application module 20 can also be provided on any software storage medium such as a floppy disk, CD, DVD, etc. as discussed above.

In an exemplary embodiment as depicted in FIG. 1, application module 20 includes various software modules for processing data content. A communication interface module 22 is provided for communicating text and/or other data to a display driver for rendering images (e.g., GUI images) on display 38, and for communicating with device/server 32 and/or other computers or server systems in network embodiments. A user interface module 24 is provided for receiving user input, commands, and signals from user interface device 36. Communication interface module 22 preferably includes a browser application, which may be the same browser as the default browser configured on client system 10 as described previously, or any other browser or user interface application. Alternatively, interface module 22 includes the functionality to interface with a browser application executing on client system 10.

Application module 20 also includes a clinical diagnostic process module 28 that performs instructions to process data according to one or more predefined clinical diagnostic processes. For example, the clinical diagnostic process may implement a complete hematology analyzer, a specific glucose analyzer, or any other clinical analytical or diagnostic process, or any variations or combinations of those or other processes. In addition, application module 20 may include other modules operable to perform other clinical diagnostic processes or analyses or quality control processes. As will be explained in more detail below, application module 20 further includes a detection module 26 operable to detect large out-of-control conditions on any or all of the clinical diagnostic process operating in the application module.

Note that while the detection module 26 is shown as operating in conjunction with the application module 20 and in conjunction with the clinical diagnostic process 28 (or processes) executing within that module, it should be understood that the detection module is not necessarily itself a part of the application process, but may operate independently of that process. Thus, while the module embodying the detection process of the present invention may be included in an instrument or system implementing a clinical diagnostic process and may execute on a system in conjunction with that process (as depicted in the exemplary system of FIG. 1), or may even be coded into a single executable application with that process, detection of large out-of-control conditions process of the present invention may also be used or implemented in conjunction with other clinical diagnostic processes or in a stand-alone configuration, that is contemplated by and within the scope of the present invention.

Compiled statistics (e.g., device and instrument information), patient information, and other information are preferably stored in database 46, which may reside in memory 48, in a memory card or other memory or storage system such as an attached storage subsystem RAID drive system, for retrieval by the clinical diagnostic process module 28, the detection module 26, and other parts of application module 20. It should be appreciated that application module 20, or portions thereof, as well as appropriate data can be downloaded to and executed on client system 10.

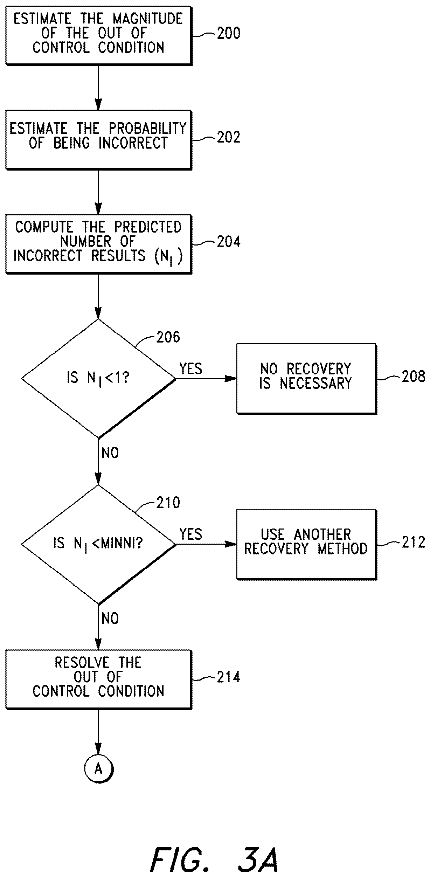

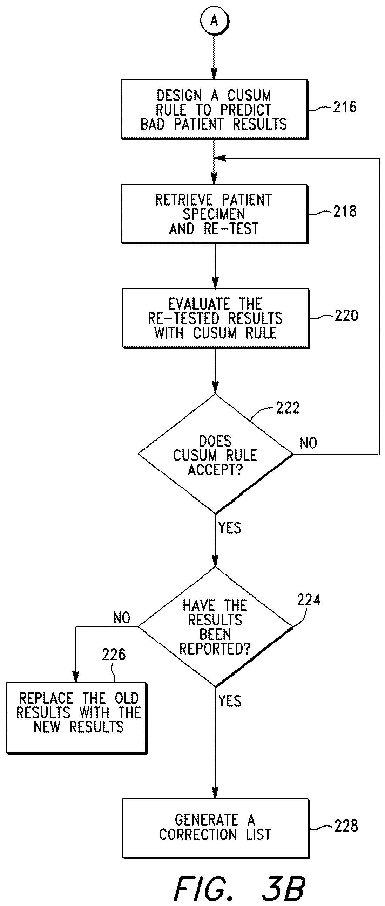

The operation of detection module 26 will now be described with particular reference to FIGS. 3A and 3B, depicting a flow diagram of an exemplary embodiment of the process for use with an individual clinical diagnostic process or with a group of clinical diagnostic processes, such as in a multi-instrument or multi-laboratory environment.

The method of the present invention automatically detects large out-of-control conditions, estimates the magnitude of the out-of-control condition, estimates the probability of a result being incorrect, and predicts the number of incorrect patient specimen results that have been generated since the last known good quality control evaluation. The method further constructs a CUSUM rule to apply to re-evaluated test results to ascertain the origination point of the large out-of-control condition. Corrections to reevaluated specimens are thus required only when warranted. In operation, a laboratory will typically implement and run a plurality of clinical diagnostic processes simultaneously, with numerous instruments in operation at any given time, with each of those instruments either running distinct clinical diagnostic processes or, as is often the case, several instruments running the same process.

The method for detecting and correcting large out-of-control conditions, or automated sequential recovery, is briefly described as follows: upon detection of an out-of-control condition, the specimen evaluation data that has been collected by a laboratory instrument is analyzed to determine the scope of the potential error in that data. Analysis of that data involves estimating the magnitude of the out-of-control condition; estimating, for every specimen evaluated since the last known good condition, the probability of the specimen data being incorrect; and computing a predicted number of incorrect results ($N_I$) expected within that specimen data. If the estimated magnitude of the out-of-control condition is found to be too low (e.g., the magnitude falls short of a predetermined threshold) then no further evaluation is required because the error is within an allowable tolerance. Similarly, if the predicted number of incorrect results ($N_I$) is less than 1 then no recovery is needed (i.e., there are no predicted incorrect results). If the predicted number of incorrect results ($N_I$) is below a predefined threshold for automated sequential recovery, then the present method for recovering from a large out-of-control condition is not suitable and another method of recovery should be employed.

If neither of those exceptions apply (i.e., $N_I$ is not less than 1 and $N_I$ is greater than a predefined threshold for automated sequential recovery), the method for recovering from a large out-of-control condition is applied as will now be described.

With the out-of-control condition corrected, using the estimated magnitude of the out-of-control condition previously calculated, a CUSUM rule is designed for detecting if a patient result was produced during the time the out-of-control condition existed. Starting with the last patient specimen tested prior to the detection of the out-of-control condition, each specimen is retested and the difference between the retested specimen result and the original specimen result is evaluated using the designed CUSUM rule. When either: (1) the CUSUM rule acceptance limits are not met (i.e., the origination point of the occurrence of the large out-of-control condition has been identified) or (2) testing of the first patient specimen tested since the last good QC evaluation occurs (i.e., all specimens since the last know good QC state have been retested), the automatic retesting of the patient specimens is stopped.

For each patient specimen re-tested/re-evaluated, if the initial results (i.e., the evaluation of the specimens prior to detection of the out-of-control condition) have been published or reported, then a correction list having the results of the re-evaluation of the specimens is generated. If the initial results have not been published, then the initial results are replaced by the results of the re-evaluation of the patient specimens.

Referring now to FIGS. 3A and 3B, a flow diagram of an exemplary embodiment of the large out-of-control condition detection and correction method of the present invention is depicted. For each clinical diagnostic processes with which the system and method of the present invention is to be used, a laboratory must initially define several process parameters as follows:

NUMREP - is the number of replicates at each quality control (QC) concentration level to be used to estimate the size of the out-of-control condition. Typically NUMREP values will preferably be in the range of 2 to 4, although values outside of that range may be appropriate depending on the specific clinical diagnostic processes and/or specific analytes.

$TE_a$ - is the allowable total error for the clinical diagnostic process. The value of $TE_a$ is typically determined by the operator of the laboratory, preferably in consideration of the technical capability and the clinical utility of the analyte. $TE_a$ is preferably based as a percentage or based on a function that assigns a value for $TE_a$ based on a concentration of the analyte.

MINNI - is the minimum predicted number of incorrect results for the applicability of the automated sequential recovery method of the present invention. If the predicted number of incorrect results is under this limit, then other recovery procedures should be used. MINNI is preferably selected based on the number of patient specimens that are evaluated between quality control (QC) events. As will be explained in more detail below, if the predicted number of incorrect results is less than MINNI, then the automated sequential recovery of the present invention may not be suitable and other recovery procedures may be used.

Preferably, a laboratory operator will determine default or initial values of NUMREP, $TE_a$, and MINNI based on the laboratory's history and knowledge of the various clinical diagnostic processes being implemented and the type of analytes being processed. Of course, as the laboratory's knowledge base increases, adjustments or changes to the initial or default values may be implemented as necessary within the scope of the present invention.

With the initial parameters for a particular clinical diagnostic process defined and set-up as just described, the general process steps for implementing the automatic detection and correction of large out-of-control conditions of the present invention are depicted in the flow diagram of FIGS. 3A and 3B. Each step will first be described generally; a more detailed description of each step in the process is set forth below.

Looking to FIGS. 3A and 3B, when an out-of-control condition is identified, at block 200 an estimate of the magnitude of the out-of-control condition is made across the concentration range of the patient results.

At block 202, for every specimen evaluated since the last good quality control evaluation, the probability of that evaluation being incorrect is estimated.

At block 204, the predicted number of incorrect results ($N_I$) evaluated since the last good quality control evaluation is computed.

At block 206, if the calculated predicted number of incorrect results ($N_I$) is less than one, then no recovery is necessary and the clinical diagnostic process may continue at block 208. At block 210, if the calculated predicted number of incorrect results is less than a predefined threshold, MINNI (minimum predicted incorrect results for automated sequential recovery, as defined above), then the automated sequential recovery of the present invention is not appropriate.

In that case, another recovery method should be used and the process terminates at block 212. As discussed above, the automated sequential recovery process of the present invention is suitable for use with large out-of-control conditions. If the out-of-control condition is not large, then another method of recovery will likely be more suitable. For example, other methods such as simply re-testing every specimen since the last good QC event will be apparent to those skilled in the art and are discussed above.

If the predicted number of incorrect results is greater that the MINNI threshold, then the process continues at block 214 where the out of control condition is resolved. It will be apparent to one skilled in the art that the potential causes of out-of-control conditions are numerous and the required actions to resolve the malfunction are varied.

With the out-of-control condition resolved, at block 216 a CUSUM rule (as will be discussed in more detail, below) is designed to identify bad patient testing results from the testing already completed. At block 218 the already-tested patient specimens are re-tested or re-evaluated, starting with the last specimen tested before detection of the large out-of-control condition, generating new results for each of those specimens.

At block 220 the designed CUSUM rule is applied to each re-tested specimen and at block 222, if the CUSUM rule accepts the reevaluated specimen result, then the point of origin of the occurrence of the large out-of-control condition has not yet been identified, and re-testing of patient specimens continues at block 218. If the CUSUM rule rejects the reevaluated specimen result, that indicates that the original test result was not tainted by the large out-of-control condition, thus identifying the point of origin of the large out-of-control condition. Re-testing of patient specimens is thus completed, and the process continues at block 224.

At block 224, it is determined whether the initial results for the patient specimens re-tested were already reported or published. If the results were not reported, then at block 226 the old results are replaced with the new results.

If the results were already reported, then at block 228 a correction list is generated having the results of any specimen where: (1) the new result is on a different side of a medical decision limit from the old result (e.g., the new result has changed the outcome of the medical decision) or (2) the new result differs from the old result by more than the allowable total error $T_{EA}$ (e.g., the new result varies from the old result by more than an allowable amount).

Thus, the automated sequential recovery process of the present invention allows controlled re-testing of specimens, identifying the point of origin of the large out-of-control condition and re-testing only those specimens affected by the out-of-control condition.

With the process set forth generally, each of the above-described steps will now be described in more detail, still with reference to FIGS. 3A and 3B.

At block 200, the magnitude of the out-of-control condition is evaluated as follows:

First, a selected number of quality control (QC) specimens are evaluated for each QC level currently being tested by the clinical diagnostic process. Preferably, the selected number of quality control specimens is NUMREP, as initially set-up as described previously.

Next, using the results of the QC evaluation just performed, a bias function is constructed for each concentration $x$ of QC being considered as:

$$bias(x) = MEAN(QC\ result) - MEAN\ (QC\ target\ level)$$

It should be understood that this calculation should include the values of QC results from the QC event result that failed.

For concentrations $x$ that fall between QC levels, the bias is calculated by linearly interpolating between those QC levels. For concentrations above or below QC levels the bias is truncated such that for concentrations above the highest level of QC, the bias of the highest level of QC is used and for concentrations below the lowest level of QC, the bias level of the lowest level of QC is used.

Next, using historic QC results, an imprecision function is constructed for each concentration $x$ of QC being considered as:

$$SD(x) = SD(QC_{target\ level})$$

In other words, for each concentration of QC being considered, the SD of the QC target level is assigned to $SD(x)$.

In cases where the concentrations that fall between QC levels, the $SD$ is calculated by linearly interpolating between those QC levels. For concentrations above or below QC levels the $SD$ is truncated such that for concentrations above the highest level of QC, the $SD$ of the highest level of QC is used; and for concentrations below the lowest level of QC, the $SD$ level of the lowest level of QC is used.

Thus, for each value in the patient data, the concentration of that patient sample is compared to the concentrations in the specimen tuples (concentration, $SD$) for the laboratory instrument on which the patient value was obtained.

When there is a direct match between the patient concentration and a concentration in the tuples, the corresponding *SD* from the tuple is assigned to that patient sample. Note that a direct match may be considered either an exact match of concentrations, or a match within a predetermined threshold (e.g., if the patient concentration is within 0.1 percent of the concentration in the specimen data). When a patient concentration does not directly match any of concentrations represented in the tuples, the *SD*'s for the patient data are calculated by interpolating as just described above and truncated when the *SD*'s exceed upper or lower thresholds.

In an alternative exemplary embodiment, the *SD*'s for the patient data are calculated by interpolating or extrapolating from the *SD*'s in the tuples as follows:

When a patient concentration falls between the concentrations of two tuples, (where the lower concentration tuple is designated ($conc_0$ $SD_0$) and the higher concentration tuple is designated ($conc_1$, $SD_1$) the *SD* for the patient data ($SD_p$) is calculated as $SD_p = SD_0 + (\text{concentration} - conc_0) * (SD_1 - SD_0) / (conc_1 - conc_0)$

When a patient concentration falls below the lowest (concentration, *SD*) tuple, designated ($conc_\ell$ $SD_\ell$), the SD value of that lowest concentration tuple is assigned to the patient SD, as $SD_p = SD_\ell$

Finally, when a patient concentration is higher than the highest (concentration, SD) tuple, designated ($conc_h$, $SD_h$) the value of the assigned patient SD is calculated as $SD_p = (SD_h / conc_h) * (\text{patient concentration})$.

Thus, for each patient value, a SD is assigned based on either a direct match with the specimen data concentrations, by interpolating between specimen values as described above, or by extrapolating from specimen values. It should be understood that while a simple piecewise linear interpolation function and lower limit truncation function have been described; other interpolation and extrapolation schemes may of course be implemented in accordance with the present invention.

Next, for every analyte concentration, *x*, the assigned $TE_a$ (as described above) is used to construct $TE_a(x)$. In the case where $TE_a$ is given as a percentage, this value may be deminimus, e.g., in cases where $TE_a(x) = \%TE_a*x/100$.

An estimated Relative Error (RE) function is defined for each analyte concentration, *x*, for each of the NUMREP QC evaluations, using the *bias(x)* and *SD(x)* functions just described, as:

$$RE(x) = bias(x)/(\sqrt{2} *SD(x))$$

Finally, an average Relative Error (AVERAGE_RE) is computed by averaging the values of the RE(x) function, as:

$$AVERAGE_{RE} = MEAN\ (RE(x))$$

With the size of the out-of-control condition estimated, at block 202, for every specimen *x* evaluated since the last good quality control evaluation, the probability of that evaluation being incorrect (*pI(x)*) is estimated as follows:

$$pI(x) = 1 - normcdf\ (TE_a(x),\ bias(x),\ sd(x)) + normcdf(-TE_a(x),\ bias(x),\ sd(x))$$

Where $TE_a(x)$, *bias(x)*, and *sd(x)* are the functions described above with respect to block 200, and where *normcdf* is the normal cumulative distribution function:

$$normcdf(x,\mu,\sigma) = \frac{1}{\sigma\sqrt{2\pi}} \int_{-\infty}^{x} e^{\frac{-(t-\mu)^2}{2\sigma^2}} dt$$

Where $\mu$ is the mean of the distribution for which the normal cumulative distribution is being computed (i.e., in this case, the bias) and $\sigma$ is the standard deviation of the distribution for which the normal cumulative distribution is being computed, and where *x* is the value for which the normal cumulative distribution is being computed.

With the probability of an evaluation being incorrect calculated, we move to block 204 where the expected number of incorrect results ($N_I$) evaluated since the last good quality control evaluation is computed as follows:

Assuming that the out-of-control condition has existed since the last good quality control evaluation (i.e., worst case analysis), then the predicted number of incorrect results can be calculated as the sum of the probabilities of any given result being incorrect for all of the patient specimens tested since the last good QC evaluation, or:

$$N_I = \sum_{i=1}^{NB} pI_i,$$

Where NB is the number of patient specimens tested since the last good QC evaluation.

At block 206, if $N_I$ is less than 1 (i.e., the estimated number of incorrect results is near zero), then no recovery is necessary and the process can continue at block 208.

At block 210, if $N_I$ is less than MINNI (as initially defined and described above), then the magnitude of the out-of-control condition is too small, and the recovery method of the present invention is not suitable. Thus, at block 212 another method of recovery must be employed.

At block 214, the problem that caused the out-of-control condition is resolved. One skilled in the art will recognize that the potential causes of out-of-control conditions are numerous. Various testing and resolution processes known in the art may be employed to troubleshoot and isolate the cause of any particular error, resolution may require test system maintenance, calibration, changing reagents, or various other actions to identify and resolve malfunctions known in the art.

Turning to FIG. 3B, with the out-of-control condition resolved, the process continues at block 216 where a CUSUM (cumulative sum control chart) rule is designed to evaluate and detect bad results in the patient data. The CUSUM rule is preferably designed as follows:

First, using the AVERAGE_RE as described and calculated above, a reference value, $K$, is calculated as:

$$K = AVERAGE\_RE / 2$$

Then, the expected number of evaluations to false rejection, $E(Nfr)$ is calculated as:

$$E(Nfr) = NB*2$$

Where NB is the number of patient specimens tested since the last good QC evaluation.

Next, using the values for $K$ and $E(Nfr)$ just calculated, a value for a decision interval, $H$, is determined from the lookup table shown in TABLE 1, with $E(Nfr)$ indexing a row in the table and $K$ indexing a column in the table.

TABLE 1

| E(Nfr)/K | 0.1 | 0.2 | 0.3 | 0.4 | 0.5 | 0.6 | 0.7 | 0.8 | 0.9 | 1 | 1.1 | 1.2 | 1.3 | 1.4 | 1.5 |
|---|---|---|---|---|---|---|---|---|---|---|---|---|---|---|---|
| 10 | 2.73 | 2.29 | 1.95 | 1.68 | 1.46 | 1.27 | 1.10 | 0.95 | 0.82 | 0.69 | 0.57 | 0.46 | 0.35 | 0.25 | 0.15 |
| 20 | 4.07 | 3.31 | 2.77 | 2.35 | 2.03 | 1.77 | 1.56 | 1.37 | 1.21 | 1.07 | 0.93 | 0.81 | 0.69 | 0.58 | 0.47 |
| 30 | 5.00 | 3.99 | 3.29 | 2.78 | 2.39 | 2.08 | 1.82 | 1.61 | 1.43 | 1.27 | 1.13 | 1.00 | 0.88 | 0.76 | 0.65 |
| 40 | 5.74 | 4.51 | 3.68 | 3.09 | 2.64 | 2.30 | 2.02 | 1.79 | 1.59 | 1.42 | 1.27 | 1.13 | 1.00 | 0.88 | 0.77 |
| 50 | 6.36 | 4.93 | 4.00 | 3.34 | 2.85 | 2.47 | 2.17 | 1.92 | 1.71 | 1.53 | 1.37 | 1.23 | 1.10 | 0.98 | 0.86 |
| 60 | 6.89 | 5.28 | 4.26 | 3.55 | 3.02 | 2.61 | 2.29 | 2.03 | 1.81 | 1.62 | 1.46 | 1.31 | 1.18 | 1.05 | 0.93 |
| 70 | 7.36 | 5.59 | 4.48 | 3.72 | 3.16 | 2.73 | 2.40 | 2.12 | 1.89 | 1.70 | 1.53 | 1.38 | 1.24 | 1.11 | 0.99 |
| 80 | 7.79 | 5.86 | 4.68 | 3.88 | 3.29 | 2.84 | 2.49 | 2.20 | 1.96 | 1.76 | 1.59 | 1.44 | 1.30 | 1.17 | 1.05 |
| 90 | 8.17 | 6.10 | 4.86 | 4.01 | 3.40 | 2.94 | 2.57 | 2.27 | 2.03 | 1.82 | 1.64 | 1.49 | 1.34 | 1.21 | 1.09 |
| 100 | 8.52 | 6.32 | 5.01 | 4.14 | 3.50 | 3.02 | 2.64 | 2.34 | 2.09 | 1.87 | 1.69 | 1.53 | 1.39 | 1.25 | 1.13 |
| 150 | 9.94 | 7.20 | 5.64 | 4.62 | 3.89 | 3.35 | 2.92 | 2.58 | 2.30 | 2.07 | 1.87 | 1.70 | 1.55 | 1.41 | 1.28 |
| 200 | 11.02 | 7.84 | 6.08 | 4.96 | 4.17 | 3.58 | 3.13 | 2.76 | 2.46 | 2.21 | 2.00 | 1.82 | 1.66 | 1.52 | 1.39 |
| 250 | 11.89 | 8.34 | 6.44 | 5.23 | 4.39 | 3.77 | 3.28 | 2.90 | 2.58 | 2.32 | 2.10 | 1.92 | 1.75 | 1.60 | 1.47 |
| 300 | 12.62 | 8.76 | 6.73 | 5.45 | 4.57 | 3.92 | 3.41 | 3.01 | 2.68 | 2.41 | 2.19 | 1.99 | 1.82 | 1.67 | 1.53 |
| 350 | 13.25 | 9.12 | 6.97 | 5.64 | 4.72 | 4.04 | 3.52 | 3.11 | 2.77 | 2.49 | 2.25 | 2.05 | 1.88 | 1.73 | 1.58 |
| 400 | 13.81 | 9.43 | 7.19 | 5.80 | 4.85 | 4.15 | 3.62 | 3.19 | 2.84 | 2.55 | 2.31 | 2.11 | 1.93 | 1.77 | 1.63 |
| 450 | 14.31 | 9.71 | 7.38 | 5.95 | 4.97 | 4.25 | 3.70 | 3.26 | 2.91 | 2.61 | 2.37 | 2.16 | 1.98 | 1.82 | 1.67 |
| 500 | 14.76 | 9.96 | 7.55 | 6.08 | 5.07 | 4.34 | 3.77 | 3.33 | 2.96 | 2.67 | 2.41 | 2.20 | 2.02 | 1.85 | 1.71 |
| 550 | 15.18 | 10.18 | 7.70 | 6.19 | 5.16 | 4.41 | 3.84 | 3.39 | 3.02 | 2.71 | 2.46 | 2.24 | 2.05 | 1.89 | 1.74 |
| 600 | 15.56 | 10.39 | 7.84 | 6.30 | 5.25 | 4.49 | 3.90 | 3.44 | 3.07 | 2.76 | 2.50 | 2.28 | 2.09 | 1.92 | 1.77 |
| 650 | 15.91 | 10.58 | 7.98 | 6.40 | 5.33 | 4.55 | 3.96 | 3.49 | 3.11 | 2.80 | 2.53 | 2.31 | 2.12 | 1.95 | 1.80 |
| 700 | 16.24 | 10.76 | 8.10 | 6.49 | 5.40 | 4.61 | 4.01 | 3.54 | 3.15 | 2.83 | 2.56 | 2.34 | 2.14 | 1.97 | 1.82 |
| 750 | 16.54 | 10.92 | 8.21 | 6.58 | 5.47 | 4.67 | 4.06 | 3.58 | 3.19 | 2.87 | 2.60 | 2.37 | 2.17 | 2.00 | 1.85 |
| 800 | 16.83 | 11.08 | 8.31 | 6.65 | 5.54 | 4.72 | 4.11 | 3.62 | 3.22 | 2.90 | 2.62 | 2.39 | 2.19 | 2.02 | 1.87 |
| 850 | 17.11 | 11.22 | 8.41 | 6.73 | 5.60 | 4.78 | 4.15 | 3.66 | 3.26 | 2.93 | 2.65 | 2.42 | 2.22 | 2.04 | 1.89 |
| 900 | 17.37 | 11.36 | 8.51 | 6.80 | 5.65 | 4.82 | 4.19 | 3.69 | 3.29 | 2.96 | 2.68 | 2.44 | 2.24 | 2.06 | 1.91 |

(continued)

| E(Nfr)/K | 0.1 | 0.2 | 0.3 | 0.4 | 0.5 | 0.6 | 0.7 | 0.8 | 0.9 | 1 | 1.1 | 1.2 | 1.3 | 1.4 | 1.5 |
|---|---|---|---|---|---|---|---|---|---|---|---|---|---|---|---|
| 950 | 17.61 | 11.49 | 8.60 | 6.87 | 5.71 | 4.87 | 4.23 | 3.73 | 3.32 | 2.98 | 2.70 | 2.46 | 2.26 | 2.08 | 1.93 |
| 1000 | 17.84 | 11.62 | 8.68 | 6.93 | 5.76 | 4.91 | 4.27 | 3.76 | 3.35 | 3.01 | 2.73 | 2.49 | 2.28 | 2.10 | 1.94 |
| 1050 | 18.07 | 11.74 | 8.76 | 6.99 | 5.81 | 4.95 | 4.30 | 3.79 | 3.38 | 3.03 | 2.75 | 2.51 | 2.30 | 2.12 | 1.96 |
| 1100 | 18.28 | 11.85 | 8.84 | 7.05 | 5.85 | 4.99 | 4.33 | 3.82 | 3.40 | 3.06 | 2.77 | 2.52 | 2.32 | 2.13 | 1.97 |
| 1150 | 18.48 | 11.96 | 8.91 | 7.10 | 5.90 | 5.03 | 4.37 | 3.85 | 3.43 | 3.08 | 2.79 | 2.54 | 2.33 | 2.15 | 1.99 |
| 1200 | 18.68 | 12.06 | 8.98 | 7.16 | 5.94 | 5.06 | 4.40 | 3.87 | 3.45 | 3.10 | 2.81 | 2.56 | 2.35 | 2.17 | 2.00 |
| 1250 | 18.87 | 12.16 | 9.05 | 7.21 | 5.98 | 5.09 | 4.43 | 3.90 | 3.47 | 3.12 | 2.83 | 2.58 | 2.36 | 2.18 | 2.02 |
| 1300 | 19.05 | 12.25 | 9.11 | 7.26 | 6.02 | 5.13 | 4.45 | 3.92 | 3.49 | 3.14 | 2.84 | 2.59 | 2.38 | 2.19 | 2.03 |
| 1350 | 19.23 | 12.35 | 9.17 | 7.30 | 6.06 | 5.16 | 4.48 | 3.95 | 3.52 | 3.16 | 2.86 | 2.61 | 2.39 | 2.21 | 2.04 |
| 1400 | 19.39 | 12.44 | 9.23 | 7.35 | 6.09 | 5.19 | 4.51 | 3.97 | 3.54 | 3.18 | 2.88 | 2.62 | 2.41 | 2.22 | 2.05 |
| 1450 | 19.56 | 12.52 | 9.29 | 7.39 | 6.13 | 5.22 | 4.53 | 3.99 | 3.55 | 3.19 | 2.89 | 2.64 | 2.42 | 2.23 | 2.07 |
| 1500 | 19.72 | 12.60 | 9.35 | 7.43 | 6.16 | 5.25 | 4.55 | 4.01 | 3.57 | 3.21 | 2.91 | 2.65 | 2.43 | 2.24 | 2.08 |
| 1550 | 19.87 | 12.68 | 9.40 | 7.47 | 6.19 | 5.27 | 4.58 | 4.03 | 3.59 | 3.23 | 2.92 | 2.67 | 2.45 | 2.26 | 2.09 |
| 1600 | 20.02 | 12.76 | 9.45 | 7.51 | 6.22 | 5.30 | 4.60 | 4.05 | 3.61 | 3.24 | 2.94 | 2.68 | 2.46 | 2.27 | 2.10 |
| 1650 | 20.16 | 12.84 | 9.50 | 7.55 | 6.26 | 5.33 | 4.62 | 4.07 | 3.63 | 3.26 | 2.95 | 2.69 | 2.47 | 2.28 | 2.11 |
| 1700 | 20.30 | 12.91 | 9.55 | 7.59 | 6.29 | 5.35 | 4.64 | 4.09 | 3.64 | 3.27 | 2.97 | 2.70 | 2.48 | 2.29 | 2.12 |
| 1750 | 20.44 | 12.98 | 9.60 | 7.62 | 6.31 | 5.37 | 4.66 | 4.11 | 3.66 | 3.29 | 2.98 | 2.72 | 2.49 | 2.30 | 2.13 |
| 1800 | 20.57 | 13.05 | 9.65 | 7.66 | 6.34 | 5.40 | 4.68 | 4.13 | 3.67 | 3.30 | 2.99 | 2.73 | 2.50 | 2.31 | 2.14 |
| 1850 | 20.70 | 13.12 | 9.69 | 7.69 | 6.37 | 5.42 | 4.70 | 4.14 | 3.69 | 3.32 | 3.00 | 2.74 | 2.51 | 2.32 | 2.15 |
| 1900 | 20.83 | 13.18 | 9.74 | 7.73 | 6.40 | 5.44 | 4.72 | 4.16 | 3.70 | 3.33 | 3.02 | 2.75 | 2.52 | 2.33 | 2.16 |
| 1950 | 20.95 | 13.25 | 9.78 | 7.76 | 6.42 | 5.46 | 4.74 | 4.18 | 3.72 | 3.34 | 3.03 | 2.76 | 2.53 | 2.34 | 2.16 |
| 2000 | 21.07 | 13.31 | 9.82 | 7.79 | 6.45 | 5.49 | 4.76 | 4.19 | 3.73 | 3.36 | 3.04 | 2.77 | 2.54 | 2.34 | 2.17 |

Thus, for example, if $E(Nfr)$ = 10 and $K$=0.5, then the value for $H$ selected from the table would be 1.46. For actual values of $K$ less than 0.1 (the smallest $K$ value in the table), the value of $K$ used is 0.1, and, for actual values of $K$ greater than 1.5 (the largest $K$ value in the table), the value of $K$ used is 1.5. Similarly, for actual values of $E(Nfr)$ falling between the values for $E(Nfr)$ shown in the table, the value used for $E(Nfr)$ is the next largest value occurring in the table. Thus, for example, if the actual calculated value for $E(Nfr)$ is 1876, then the $E(Nfr)$ used for the table lookup is 1900, the next largest value occurring in the table. Alternatively, other methods of determining a value of $H$ from the table may be used, such as interpolating between values shown in the table and/or extrapolating beyond those values.

With the decision interval, $H$, selected, the one-sided upper CUSUM ($C_U$), one-sided lower CUSUM ($C_L$) and index ($i$) parameters are initialized to a value of zero, to complete the design of the CUSUM rule.

At block 218, the patient specimens are retrieved and retested in reverse order - that is, the first specimen retested is the patient specimen tested just prior to detection of the out-of-control condition, and the last specimen retested (if necessary) is the specimen tested just after the last good QC result.

As will be used in the equations to follow, $old_x$ refers to the patient specimen result $x$ prior to detection of the out-of-control condition and $new_x$ refers to the retested specimen result $x$.

At block 220, the designed CUSUM rule is applied to the old and new results (i.e., the original and retested patient results), as follows:

First, the index (counter) value for the rule is incremented as:

$$i = i + 1.$$

Next, a composite specimen test value, $x_i$, is calculated from the $old_x$ and $new_x$ values and using the bias and SD functions previously described as:

$$x_i = ((old_x - new_x) - bias(new_x)) / (\sqrt{2}\, SD(new_x))$$

Then, the one-sided upper CUSUM ($C_U$) and one-sided lower CUSUM ($C_L$) values are calculated as:

$$C_{Ui} = max(0,[x_i - K + C_{Ui-1}])$$

$$C_{Li} = max(0,[-K - x_i + C_{Li-1}])$$

Next, at block 222, the results of the applied CUSUM rule are reviewed. If either of the calculated values of $C_U$ or $C_L$ are greater than the decision interval $H$, or if the index $i$ has reached $NB$ (i.e., all specimens since the last known good QC condition have been re-tested) then the CUSUM rule rejects and the patient specimen retesting is completed. Or, expressed mathematically,

$$If\ (C_{Ui} > H)\ or\ (C_{Li} > H),\ or\ if\ i = NB,\ then\ reject$$

At block 224 it is determined whether the results of the initial evaluation of the patient specimens have been reported or published. If not, at block 226, the initial evaluation results are replaced with the re-evaluation results produced by spot-checking and re-evaluating the patient specimens.

If the results have previously been published, then at block 228, a correction list is generated having any specimen results where the new result is on a different side of a medical decision limit from the old results, or where the new result differs from the old result by more than the allowable total error $TE_a$.

Thus, at block 228, a new result is added to the correction list if:

$$|new\ result - old\ result\ | > TE_a(new\ result)$$

Similarly, a new result is added to the correction list if the signs of the results of the following two calculations are different (i.e., if one is positive and one is negative):

$$old\ result - decision\ limit;\ and\ new\ result - decision\ limit$$

However, if the signs of the results of the two calculations are the same, the result of the re-evaluation is not added to the correction list.

Thus, upon detection of an out-of-control condition, the system and method of the present invention automatically characterizes a large out-of-control condition and predicts the number of unreliable patient specimen results since the last known good quality control evaluation. The system and method further constructs a CUSUM rule and begins re-evaluating/re-testing the specimens when the out-of-control condition has been corrected, beginning with the last specimen tested prior to detection of the out-of-control condition. The specimens are iteratively re-tested, and the CUSUM rule is applied to detect when a re-tested specimen is acceptable - i.e., identifying the point of origin of the occurrence of the out-of-control condition. At that point, re-testing of the specimens is halted, and old incorrect data results are replaced with the new, retested data only for those data points requiring correction. With the error detected and corrected, and the tainted data corrected, the clinical diagnostic process can continue. Thus, re-evaluation/re-testing of specimens occurs only as required, and only those data points needing correction are corrected, thus avoiding costly, time-consuming, and unnecessary re-testing.

Any quantitative representation presented herein which could permissibly vary without resulting in a change in the basic function to which it is related may permissibly vary from that if the variance does not materially alter the capability of the invention.

While the present invention has been described and illustrated hereinabove with reference to various exemplary embodiments, it should be understood that various modifications could be made to these embodiments without departing from the scope of the invention. Therefore, the invention is not to be limited to the exemplary embodiments described and illustrated hereinabove, except insofar as such limitations are included in the following claims.

**Claims**

1. Method for analyzing at least one specimen comprising the steps:

   analyzing at least one specimen by using at least one laboratory instrument;

quality-checking the analysis results for determining an out-of-control condition, in particular a large out-of-control condition;

upon receiving a notification of an out-of-control condition from the at least one laboratory instrument: analyzing data of a specimen test from said laboratory instrument to determine a scope of potential error caused by said out-of-control condition;

determining the scope of potential error comprising estimating the magnitude of the out of control condition (200);

estimating for every specimen evaluated since the last known good condition the probability of the specimen data being incorrect (202);

computing a predicted number of incorrect results $N_I$ expected within that specimen data (204),

whereby, if the estimated magnitude of the out of control condition is found to be too low, no further evaluation is conducted,

if the predicted number of incorrect results $N_I$ is less than 1 (206), no recovery is conducted (208),

if the predicted number of incorrect results $N_I$ is below a predefined threshold for automated sequential recovery (210), applying another recovery method (212),

if $N_I$ is not less than 1 and $N_I$ is greater than a predefined threshold for automated sequential recovery applying the following steps for recovering from a large out of control condition (214):

designing a CUSUM rule to predict if a specimen test data point was produced while the large out-of-control condition existed (216);

retrieving patient specimen and retesting them (218), thereby

re-evaluating at least a portion of previously tested specimens to generate new data of test results (220);

applying said CUSUM rule to accept or reject the re-evaluated data of test results (222);

replacing at least a portion of previously tested specimen data with said new data of test results (226).

2. The method of claim 1, wherein said designing a CUSUM rule comprises calculating an average relative error and determining a decision interval based at least in part on said average relative error.

3. The method of claim 2, wherein said decision interval is selected from a predetermined lookup table.

4. The method of claim 2 - 3, further comprising calculating an expected number or evaluations to false rejection, and wherein said determining a decision interval is based at least in part on said average relative error, said expected numbers of evaluations to false rejection, and combinations thereof.

5. The method of claim 1 - 4, wherein said applying a CUSUM rule comprises calculating a composite specimen test value, calculating a one-sided upper CUSUM value, and calculating a one-sided lower CUSUM value.

6. System for analyzing at least one specimen, the system comprising:

one or more laboratory instruments operable to analyze at least one specimen to determine an out of control condition;

a computer system operable to communicate with and receive data of test results from said at least one laboratory instrument, said computer system having a processor operable to:

receiving notification of an out-of-control condition from a laboratory instrument;

wherein the out-of-control condition has been determined by analysis of the at least one laboratory instrument and;

upon receipt of notification of an out-of-control condition from the at least one laboratory instrument,

analyze data of a specimen test from said laboratory instrument to determine a scope of potential error caused by said out-of-control condition;

determining the scope of potential error comprising estimating the magnitude of the out of control condition (200);

estimating for every specimen evaluated since the last known good condition the probability of the specimen data being incorrect (202);

computing a predicted number of incorrect results $N_I$ expected within that specimen data (204),

whereby, if the estimated magnitude of the out of control condition is found to be too low, no further evaluation is conducted,

if the predicted number of incorrect results $N_I$ is less than 1 (206), no recovery is conducted (208),

if the predicted number of incorrect results $N_I$ is below a predefined threshold for automated sequential recovery (210), applying another recovery method (212),
if $N_I$ is not less than 1 and $N_I$ is greater than a predefined threshold for automated sequential recovery applying the following steps for recovering from a large out of control condition (214):

design a CUSUM rule to predict if data of the specimen test was produced during the out-of-control condition (216);
retrieve patient specimen and retest them (218), thereby
re-evaluate at least a portion of previously tested specimens to generate new data of test results (220); and
apply said CUSUM rule to accept or reject the re-evaluated data of test results (222).

7. The system of claim 6, wherein said applying a CUSUM rule comprises calculating a composite specimen test value, calculating a one-sided upper CUSUM value, and calculating a one-sided lower CUSUM value.

8. The system of claim 7, wherein said estimating a magnitude of said out of control condition comprises evaluating quality control specimens and determining an allowable total error based on bias and historic imprecision data.

9. The system of claim 8, wherein said estimating a probability of a specimen evaluation being incorrect comprises calculating a normal cumulative distribution.

10. The system of claim 6 - 9, wherein said processor is further operable to:
replace at least a portion of said data of the specimen test with re-evaluated specimen data (226).

11. The system of claim 6 - 10, wherein said processor is further operable to:
generate a report identifying re-evaluated specimen data (228).

12. A non-transitory computer-readable medium having computer-executable instructions for performing a method for automatically detecting and correcting large out-of-control conditions in a clinical diagnostic process, the method comprising:

analyzing at least one specimen by using at least one laboratory instrument;
quality-checking the analysis results for determining an out-of-control condition, in particular a large out-of-control condition;
upon receiving a notification of an out-of-control condition from a laboratory instrument: analyzing data of a specimen test from said laboratory instrument to determine a scope of potential error caused by said out-of-control condition;
determining the scope of potential error comprising estimating the magnitude of the out of control condition (200);
estimating for every specimen evaluated since the last known good condition the probability of the specimen data being incorrect (202);
computing a predicted number of incorrect results $N_I$ expected within that specimen data (204),
whereby, if the estimated magnitude of the out of control condition is found to be too low, no further evaluation is conducted,
if the predicted number of incorrect results $N_I$ is less than 1 (206), no recovery is conducted (208),
if the predicted number of incorrect results $N_I$ is below a predefined threshold for automated sequential recovery (210), applying another recovery method (212),
if $N_I$ is not less than 1 and $N_I$ is greater than a predefined threshold for automated sequential recovery applying the following steps for recovering from a large out of control condition (214):

designing a CUSUM rule to predict if a specimen test data point was produced during the large out-of-control condition (216);
retrieving patient specimen and retesting them (218), thereby
re-evaluating at least a portion of previously tested specimens to generate new data of test results (220);
applying said CUSUM rule to accept or reject the re-evaluated data of test results (222);
replacing at least a portion of previously tested specimen data with said new data of test results (226).

**Patentansprüche**

1. Verfahren zum Analysieren mindestens einer Probe mit folgenden Schritten:

   Analysieren mindestens einer Probe unter Verwendung von mindestens einem Laborinstrument;
   Prüfen der Qualität der Analyseergebnisse zum Ermitteln eines außer Kontrolle geratenen Zustands, insbesondere eines großen außer Kontrolle geratenen Zustands unter Verwendung mindestens eines Laborinstruments;
   beim Empfangen einer Benachrichtigung über einen außer Kontrolle geratenen Zustand von dem mindestens einem Laborinstrument: Analysieren von Daten eines Probentests von dem Laborinstrument zum Ermitteln eines Umfangs eines durch den außer Kontrolle geratenen Zustand verursachten potenziellen Fehlers;
   Ermitteln des Umfangs des potenziellen Fehlers, was das Schätzen der Größe des außer Kontrolle geratenen Zustands umfasst (200);
   Schätzen der Wahrscheinlichkeit von falschen Probendaten für jede ausgewertete Probe seit dem letzten bekannten guten Zustand (202);
   Berechnen einer vorausgesagten Anzahl in den Probendaten erwarteter falscher Ergebnisse $N_I$ (204),
   wobei, wenn die geschätzte Größe des außer Kontrolle geratenen Zustands als zu niedrig empfunden wird, keine weitere Auswertung durchgeführt wird, wenn die vorausgesagte Anzahl falscher Ergebnisse $N_I$ geringer als 1 ist (206), wird keine Wiederherstellung durchgeführt (208),
   wenn die vorausgesagte Anzahl falscher Ergebnisse $N_I$ unterhalb eines vorbestimmten Schwellenwerts zur automatisierten sequenziellen Wiederherstellung liegt (210), dann Anwenden einer anderen Wiederherstellungsmethode (212),
   wenn $N_I$ nicht geringer als 1 ist und $N_I$ größer als ein vorbestimmter Schwellenwert zur automatisierten sequenziellen Wiederherstellung ist, dann Anwenden der folgenden Schritte zur Wiederherstellung aus einem großen außer Kontrolle geratenen Zustand (214):

      Entwickeln einer CUSUM-Regel, um vorauszusagen, ob während des Bestehens eines großen außer Kontrolle geratenen Zustands ein Probentestdatenpunkt erstellt wurde (216);
      Abrufen von Patientenproben und erneutes Testen der Patientenproben (218), dadurch erneutes Auswerten von zumindest einem Teil von zuvor getesteten Proben zum Erzeugen neuer Daten aus den Testergebnissen (220);
      Anwenden der CUSUM-Regel zum Akzeptieren oder Ablehnen der erneut ausgewerteten Daten von Testergebnissen (222);
      Ersetzen von zumindest einem Teil zuvor getesteter Probendaten durch die neuen Daten der Testergebnisse (226).

2. Verfahren nach Anspruch 1, wobei das Entwickeln einer CUSUM-Regel das Berechnen eines mittleren relativen Fehlers und das Ermitteln eines Entscheidungsintervalls zumindest teilweise basierend auf dem mittleren relativen Fehler umfasst.

3. Verfahren nach Anspruch 2, wobei das Entscheidungsintervall aus einer vorbestimmten Lookup-Tabelle ausgewählt wird.

4. Verfahren nach Anspruch 2-3, ferner umfassend das Berechnen einer erwarteten Anzahl von Auswertungen zu falscher Ablehnung und wobei das Ermitteln eines Entscheidungsintervalls zumindest teilweise auf dem mittleren relativen Fehler, der erwarteten Anzahl von Auswertungen zu falscher Ablehnung und Kombinationen davon basiert.

5. Verfahren nach Anspruch 1-4, wobei das Anwenden einer CUSUM-Regel das Berechnen eines Verbundprobentestwerts, das Berechnen eines einseitigen oberen CUSUM-Werts und das Berechnen eines einseitigen unter CUSUM-Werts umfasst.

6. System zum Analysieren mindestens einer Probe, wobei das System aufweist:

   ein oder mehr Laborinstrumente, die zum Analysieren mindestens einer Probe zum Ermitteln eines außer Kontrolle geratenen Zustands bedienbar sind;
   ein Computersystem, das zum Kommunizieren mit und Empfangen von Daten von Testergebnisse von dem mindestens einen Laborinstrument bedienbar ist, wobei das Computersystem einen Prozessor aufweist, der betreibbar ist zum:

Empfangen einer Benachrichtigung eines außer Kontrolle geratenen Zustands von dem mindestens einen Laborinstrument;

wobei der außer Kontrolle geratene Zustand durch Analyse des mindestens einen Laborinstruments ermittelt wurde; und

beim Empfangen der Benachrichtigung eines außer Kontrolle geratenen Zustands von dem mindestens einen Laborinstrument,

Analysieren von Daten eines Probentests von dem Laborinstrument zum Ermitteln eines Umfangs eines durch den außer Kontrolle geratenen Zustand verursachten potenziellen Fehlers;

Ermitteln des Umfangs des potenziellen Fehlers, was das Schätzen der Größe des außer Kontrolle geratenen Zustands umfasst (200);

Schätzen der Wahrscheinlichkeit von falschen Probendaten für jede ausgewertete Probe seit dem letzten bekannten guten Zustand (202);

Berechnen einer vorausgesagten Anzahl in den Probendaten erwarteter falscher Ergebnisse $N_l$ (204),

wobei, wenn die geschätzte Größe des außer Kontrolle geratenen Zustands als zu niedrig empfunden wird, wird keine weitere Auswertung durchgeführt, wenn die vorausgesagte Anzahl falscher Ergebnisse $N_l$ geringer als 1 ist (206), wird keine Wiederherstellung durchgeführt (208),

wenn die vorausgesagte Anzahl falscher Ergebnisse $N_l$ unterhalb eines vorbestimmten Schwellenwerts zur automatisierten sequenziellen Wiederherstellung liegt (210), dann Anwenden einer anderen Wiederherstellungsmethode (212),

wenn $N_l$ nicht geringer als 1 ist und $N_l$ größer als ein vorbestimmter Schwellenwert zur automatisierten sequenziellen Wiederherstellung ist, dann Anwenden der folgenden Schritte zur Wiederherstellung aus einem großen außer Kontrolle geratenen Zustand (214):

Entwickeln einer CUSUM-Regel, um vorauszusagen, ob während des Bestehens eines großen außer Kontrolle geratenen Zustands ein Probentestdatenpunkt erstellt wurde (216);

Abrufen von Patientenproben und erneutes Testen der Patientenproben (218), dadurch

erneutes Auswerten von zumindest einem Teil von zuvor getesteten Proben zum Erzeugen neuer Daten aus den Testergebnissen (220); und

Anwenden der CUSUM-Regel zum Akzeptieren oder Ablehnen der erneut ausgewerteten Daten von Testergebnissen (222).

**7.** System nach Anspruch 6, wobei das Anwenden einer CUSUM-Regel das Berechnen eines Verbundprobentestwerts, das Berechnen eines einseitigen oberen CUSUM-Werts und das Berechnen eines einseitigen unter CUSUM-Werts umfasst.

**8.** System nach Anspruch 7, wobei das Schätzen einer Größe des außer Kontrolle geratenen Zustands das Schätzen von Qualitätskontrollproben und das Ermitteln eines zulässigen Gesamtfehlers basierend auf Verzerrungsdaten und historischen Ungenauigkeitsdaten umfasst.

**9.** System nach Anspruch 8, wobei das Schätzen einer Wahrscheinlichkeit einer falschen Probenauswertung das Berechnen einer normalen kumulativen Verteilung umfasst.

**10.** System nach Anspruch 6-9, wobei der Prozessor ferner betreibbar ist zum:
Ersetzen von zumindest einem Teil der Daten des Probentests mit erneut ausgewerteten Probendaten (226).

**11.** System nach Anspruch 6-10, wobei der Prozessor ferner betreibbar ist zum:
Erzeugen eines Berichts, der erneut ausgewertete Probendaten identifiziert (228).

**12.** Nicht vorübergehendes computerlesbares Medium mit computerausführbaren Anweisungen zum Durchführen eines Verfahrens zum automatischen Detektieren und Korrigieren großer außer Kontrolle geratener Zustände in einem klinischen Diagnoseprozess, wobei das Verfahren umfasst:

Analysieren von mindestens einer Probe unter Verwendung von mindestens einem Laborinstrument;

Prüfen der Qualität der Analyseergebnisse zum Ermitteln eines außer Kontrolle geratenen Zustands, insbesondere eines großen außer Kontrolle geratenen Zustands;

beim Empfangen einer Benachrichtigung über eine außer Kontrolle geratenen Zustand von einem Laborinstrument: Analysieren von Daten eines Probentests von dem Laborinstrument zum Ermitteln eines Umfangs eines durch den außer Kontrolle geratenen Zustand verursachten potenziellen Fehlers;

Ermitteln des Umfangs des potenziellen Fehlers, was das Schätzen der Größe des außer Kontrolle geratenen Zustands umfasst (200);

Schätzen der Wahrscheinlichkeit von falschen Probendaten für jede ausgewertete Probe seit dem letzten bekannten guten Zustand (202);

Berechnen einer vorausgesagten Anzahl in den Probendaten erwarteter falscher Ergebnisse $N_I$ (204), wobei, wenn die geschätzte Größe des außer Kontrolle geratenen Zustands als zu niedrig empfunden wird, wird keine weitere Auswertung durchgeführt, wenn die vorausgesagte Anzahl falscher Ergebnisse $N_I$ geringer als 1 ist (206), wird keine Wiederherstellung durchgeführt (208),

wenn die vorausgesagte Anzahl falscher Ergebnisse $N_I$ unterhalb eines vorbestimmten Schwellenwerts zur automatisierten sequenziellen Wiederherstellung liegt (210), dann Anwenden einer anderen Wiederherstellungsmethode (212),

wenn $N_I$ nicht geringer als 1 ist und $N_I$ größer als ein vorbestimmter Schwellenwert zur automatisierten sequenziellen Wiederherstellung ist, dann Anwenden der folgenden Schritte zur Wiederherstellung aus einem großen außer Kontrolle geratenen Zustand (214):

Entwickeln einer CUSUM-Regel, um vorauszusagen, ob während des Bestehens eines großen außer Kontrolle geratenen Zustands ein Probentestdatenpunkt erstellt wurde (216);

Abrufen von Patientenproben und erneutes Testen der Patientenproben (218), dadurch

erneutes Auswerten von zumindest einem Teil von zuvor getesteten Proben zum Erzeugen neuer Daten aus den Testergebnissen (220);

Anwenden der CUSUM-Regel zum Akzeptieren oder Ablehnen der erneut ausgewerteten Daten von Testergebnissen (222);

Ersetzen von zumindest einem Teil zuvor getesteter Probendaten durch die neuen Daten der Testergebnisse (226).

## Revendications

1. Procédé d'analyse d'au moins une éprouvette comprenant les étapes suivantes :

l'analyse d'au moins une éprouvette en utilisant au moins un instrument de laboratoire ;

la réalisation d'une vérification de qualité des résultats d'analyse pour déterminer un état hors de contrôle, en particulier un état hors de contrôle de grande envergure ;

à la réception d'une notification d'un état hors de contrôle provenant de l'au moins un instrument de laboratoire :

l'analyse de données d'un essai d'éprouvette provenant dudit instrument de laboratoire pour déterminer une portée d'erreur potentielle causée par ledit état hors de contrôle ;

la détermination de la portée d'erreur potentielle comprenant une estimation de l'ampleur de l'état hors de contrôle (200) ;

l'estimation, pour chaque éprouvette évaluée depuis le dernier bon état connu, de la probabilité pour les données d'éprouvette d'être incorrectes (202) ;

le calcul informatique d'un nombre prédit de résultats incorrects $N_I$ prévus dans ces données d'éprouvette (204), moyennant quoi, si l'ampleur estimée de l'état hors de contrôle s'avère trop faible, aucune évaluation n'est conduite,

si le nombre prédit de résultats incorrects $N_I$ est inférieur à 1 (206), aucune récupération n'est conduite (208),

si le nombre prédit de résultats incorrects $N_I$ est en dessous d'un seuil prédéfini pour une récupération séquentielle automatisée (210), l'application d'un autre procédé de récupération (212),

si $N_I$ n'est pas inférieur à 1 et $N_I$ est supérieur à un seuil prédéfini pour une récupération séquentielle automatisée, l'application des étapes suivantes pour une récupération d'un état hors de contrôle de grande envergure (214) :

la conception d'une règle CUSUM pour prédire si un point de données d'essai d'éprouvette a été produit au cours de l'existence de l'état hors de contrôle de grande envergure (216) ;

la récupération des éprouvettes de patients et leur soumission à un nouvel essai (218), de ce fait

la réévaluation d'au moins une portion des éprouvettes mises à l'essai précédemment afin de générer de nouvelles données de résultats d'essai (220) ;

l'application de ladite règle CUSUM pour accepter ou rejeter les données réévaluées de résultats d'essai (222) ;

le remplacement de l'au moins une portion de données d'éprouvette mises à l'essai précédemment par lesdites nouvelles données de résultats d'essai (226).

**2.** Procédé selon la revendication 1, dans lequel ladite conception d'une règle CUSUM comprend le calcul d'une erreur relative moyenne et la détermination d'un intervalle de décision sur la base au moins en partie de ladite erreur relative moyenne.

**3.** Procédé selon la revendication 2, dans lequel ledit intervalle de décision est choisi à partir d'une table de consultation prédéterminée.

**4.** Procédé selon les revendications 2 et 3, comprenant en outre le calcul d'un nombre prévu ou d'évaluations donnant lieu à un faux rejet, et dans lequel ladite détermination d'un intervalle de décision est basée au moins en partie sur ladite erreur relative moyenne, lesdits nombres prévus d'évaluations donnant lieu à un faux rejet, et leurs combinaisons.

**5.** Procédé selon les revendications 1 à 4, dans lequel ladite application d'une règle CUSUM comprend le calcul d'une valeur d'essai d'éprouvette composite, le calcul d'une valeur CUSUM supérieure unilatérale, et le calcul d'une valeur CUSUM inférieure unilatérale.

**6.** Système d'analyse d'au moins une éprouvette, le système comprenant :

un ou plusieurs instruments de laboratoire capables de fonctionner pour analyser au moins une éprouvette afin de déterminer un état hors de contrôle ;
un système d'ordinateur capable de fonctionner pour communiquer avec ledit au moins un instrument de laboratoire et recevoir des données de résultats d'essai provenant de celui-ci, ledit système d'ordinateur ayant un processeur capable de fonctionner pour :

recevoir une notification d'un état hors de contrôle provenant d'un instrument de laboratoire ;
dans lequel l'état hors de contrôle a été déterminé par analyse de l'au moins un instrument de laboratoire et ;
lors de la réception d'une notification d'un état hors de contrôle provenant de l'au moins un instrument de laboratoire,
analyser des données d'un essai d'éprouvette provenant dudit instrument de laboratoire afin de déterminer une portée d'erreur potentielle causée par ledit état hors de contrôle ;
la détermination de la portée d'erreur potentielle comprenant l'estimation de l'ampleur de l'état hors de contrôle (200) ;
estimer pour chaque éprouvette évaluée depuis le dernier bon état connu la probabilité pour les données d'éprouvette d'être incorrectes (202) ;
calculer informatiquement un nombre prédit de résultats incorrects $N_I$ prévus dans ces données d'éprouvette (204),
moyennant quoi, si l'ampleur estimée de l'état hors de contrôle s'avère trop faible, aucune évaluation n'est conduite,
si le nombre prédit de résultats incorrects $N_I$ est inférieur à 1 (206), aucune récupération n'est conduite (208),
si le nombre prédit de résultats incorrects $N_I$ est en dessous d'un seuil prédéfini pour une récupération séquentielle automatisée (210), appliquer un autre procédé de récupération (212),
si $N_I$ n'est pas inférieur à 1 et $N_I$ est supérieur à un seuil prédéfini pour une récupération séquentielle automatisée, appliquer les étapes suivantes pour une récupération d'un état hors de contrôle de grande envergure (214) :

concevoir une règle CUSUM pour prédire si des données d'essai d'éprouvette ont été produites pendant l'état hors de contrôle de grande envergure (216) ;
récupérer des éprouvettes de patients et les soumettre à un nouvel essai (218), de ce fait
réévaluer au moins une portion des éprouvettes mises à l'essai précédemment afin de générer de nouvelles données de résultats d'essai (220) ; et
appliquer ladite règle CUSUM pour accepter ou rejeter les données réévaluées de résultats d'essai (222).

**7.** Système selon la revendication 6, dans lequel ladite application d'une règle CUSUM comprend le calcul d'une valeur d'essai d'éprouvette composite, le calcul d'une valeur CUSUM supérieure unilatérale, et le calcul d'une valeur CUSUM inférieure unilatérale.

**8.** Système selon la revendication 7, dans lequel ladite estimation d'une ampleur dudit état hors de contrôle comprend l'évaluation d'éprouvettes de contrôle de qualité et la détermination d'une erreur totale admissible sur la base d'un biais et d'un historique de données d'imprécision.

9. Système selon la revendication 8, dans lequel ladite estimation d'une probabilité pour une évaluation d'éprouvette d'être incorrecte comprend le calcul d'une distribution cumulative normale.

10. Système selon les revendications 6 à 9, dans lequel ledit processeur est en outre capable de fonctionner pour : remplacer au moins une portion desdites données de l'essai d'éprouvette par des données d'éprouvette réévaluées (226).

11. Système selon les revendications 6 à 10, dans lequel ledit processeur est en outre capable de fonctionner pour : générer un rapport identifiant des données d'éprouvettes réévaluées (228).

12. Support lisible par ordinateur non transitoire ayant des instructions exécutables par ordinateur destinées à réaliser un procédé de détection et de correction automatiques d'états hors de contrôle de grande envergure dans un processus de diagnostic clinique, le procédé comprenant :

l'analyse d'au moins une éprouvette en utilisant au moins un instrument de laboratoire ;
la réalisation d'une vérification de qualité des résultats d'analyse pour déterminer un état hors de contrôle, en particulier un état hors de contrôle de grande envergure ;
à la réception d'une notification d'un état hors de contrôle provenant de l'au moins un instrument de laboratoire :
l'analyse de données d'un essai d'éprouvette provenant dudit instrument de laboratoire pour déterminer une portée d'erreur potentielle causée par ledit état hors de contrôle ;
la détermination de la portée d'erreur potentielle comprenant une estimation de l'ampleur de l'état hors de contrôle (200) ;
l'estimation, pour chaque éprouvette évaluée depuis le dernier bon état connu, de la probabilité pour les données d'éprouvette d'être incorrectes (202) ;
le calcul informatique d'un nombre prédit de résultats incorrects $N_I$ prévus dans ces données d'éprouvette (204), moyennant quoi, si l'ampleur estimée de l'état hors de contrôle s'avère trop faible, aucune évaluation n'est conduite,
si le nombre prédit de résultats incorrects $N_I$ est inférieur à 1 (206), aucune récupération n'est conduite (208),
si le nombre prédit de résultats incorrects $N_I$ est en dessous d'un seuil prédéfini pour une récupération séquentielle automatisée (210), l'application d'un autre procédé de récupération (212),
si $N_I$ n'est pas inférieur à 1 et $N_I$ est supérieur à un seuil prédéfini pour une récupération séquentielle automatisée, l'application des étapes suivantes pour une récupération d'un état hors de contrôle de grande envergure (214) :

la conception d'une règle CUSUM pour prédire si un point de données d'essai d'éprouvette a été produit pendant l'état hors de contrôle de grande envergure (216) ;
la récupération des éprouvettes de patients et leur soumission à un nouvel essai (218), de ce fait
la réévaluation d'au moins une portion des éprouvettes mises à l'essai précédemment afin de générer de nouvelles données de résultats d'essai (220) ;
l'application de ladite règle CUSUM pour accepter ou rejeter les données réévaluées de résultats d'essai (222) ;
le remplacement d'au moins une portion de données d'éprouvette mises à l'essai précédemment par lesdites nouvelles données de résultats d'essai (226).

FIG. 1

FIG. 2

EP 2 980 716 B1

```
┌─────────────────────────┐
│   ESTIMATE THE MAGNITUDE │
│     OF THE OUT OF        │──200
│   CONTROL CONDITION      │
└─────────────────────────┘
             │
             ▼
┌─────────────────────────┐
│  ESTIMATE THE PROBABILITY│──202
│    OF BEING INCORRECT    │
└─────────────────────────┘
             │
             ▼
┌─────────────────────────┐
│  COMPUTE THE PREDICTED   │
│       NUMBER OF          │──204
│  INCORRECT RESULTS (N_I) │
└─────────────────────────┘
             │
             ▼
           ╱206                    ┌──────────────────┐
         ╱    ╲        YES         │   NO RECOVERY    │
        ╱ IS N_I<1? ╲ ──────────▶  │   IS NECESSARY   │──208
         ╲    ╱                    └──────────────────┘
           ╲╱
            │ NO
            ▼
           ╱210                    ┌──────────────────┐
         ╱    ╲        YES         │   USE ANOTHER    │
        ╱IS N_I<MINNI?╲ ─────────▶ │ RECOVERY METHOD  │──212
         ╲    ╱                    └──────────────────┘
           ╲╱
            │ NO
            ▼
┌─────────────────────────┐
│     RESOLVE THE          │
│       OUT OF             │──214
│   CONTROL CONDITION      │
└─────────────────────────┘
             │
             ▼
            (A)
```

# FIG. 3A

21

FIG. 3B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8099257 B
- US 2005125186 A1
- US 2012330866 A1